# EUROPEAN PATENT APPLICATION

(11) **EP 1 693 042 A2**
(43) Date of publication of application: **23.08.2006**
(21) Application number: 04787510.9
(22) Date of filing: 02.09.2004
(51) Int. Cl.: A61J 1/00

(54) **PORTABLE BOTTLE SYSTEM FOR ADMINISTERING ENEMAS**

(30) Priority: 03.09.2003 CO 3076651
(71) Applicant: Posse Espinosa, Rafael, Bogota D.C. (CO)
(72) Inventor: Posse Espinosa, Rafael, Bogota D.C. (CO)
(74) Representative: Primo de Rivera y Urquijo, Jose A.
(86) International application number: PCT/IB2004/002973
(87) International publication number: WO 2005/020871

(57) **Abstract**

The present invention relates to a portable supporting structure combined with a container for use in administering enemas using a pressure differential between the enema solution and the intestine to facilitate insertion of the former into the latter. Specifically, the portable container system for administering enemas consists of a container, a hose and a supporting structure capable of being dismantled in such a way that all parts thereof fit inside the container, whether alone or in combination.

## Description

### Object of the Invention

The present invention relates to a portable supporting structure combined with a container for use in administering enemas using a pressure differential between the enema solution and the intestine to facilitate insertion of the former into the latter.

### Field of the Invention

The present invention can be applied in the medical field and can be used in therapeutic treatments such as administering enemas to achieve a complete intestinal cleansing, necessary in complementary examinations as well as surgical interventions or in specific diseases that require their administration.

### Background of the Invention

Administering enemas is usually a common practice. These enemas are applied with a rectal probe by means of which the enema containing liquid is introduced into the patient, subsequently withdrawing said probe so that the patient can carry out the evacuation of faeces together with the enema.

The application of enemas by known devices poses various problems for both sanitary personnel and patient; these problems are determined by the escape of faeces and of the enema, giving rise to additional work for cleaning personnel and additional expenses, as well as the obvious discomfort for the patient and the development of deficient hygienic conditions.

Various devices have currently been developed for administering enemas such as that disclosed in the Vass document, US 3906948, titled "Rectal Applicator", herein illustrated as a reference only. This Vass document discloses a rectal applicator for administering an enema usually used in a child intestinal tract examination, comprising the joining of two sections with an enlargement, where the proximal side thereof is formed by spaced concentric annular edges. There are also other single use devices such as the one disclosed in the Vita Laboratories document ES 141777, titled "Single Use Enema Applicator", herein referenced only by way of illustration. This single use enema applicator device, essentially characterized in that it comprises a flexible deposit provided with an orifice for the suspension, contains the liquid medicine and incorporates a mouth from which projects a flexible duct connected to the cannula by interpositioning an elastic, laminated opening and closing clip element to inject the enema by steps, said cannula being provided with an enveloping stopper.

Examples of devices for administering enemas and improvements of these can be found in Spanish patent documents ES1029641, granted on October 27, 1995 to Echeverria, and ES1011690, granted on May 27, 1991 to Solanes.

These prior art devices require assistance so a second or more persons must unnecessarily be used for their application. Additionally, most of these prior art device structures are not capable of being dismantled and their transport is relatively delicate as well as uncomfortable. Together with the foregoing, these previous structures can not be simply assembled.

### Description of the Invention

The present invention relates to a supporting structure combined with a container for use in administering enemas using a pressure differential between the enema solution and the intestine to facilitate insertion of the former into the latter.

### Description of the Drawings

To better understand the invention and its advantages when compared to the known art, the possible illustrating and non-limiting embodiments of applying said principles are described below with the aid of the enclosed drawings.

Figure 1 shows a perspective view of the portable system for administering enemas in an assembled state.

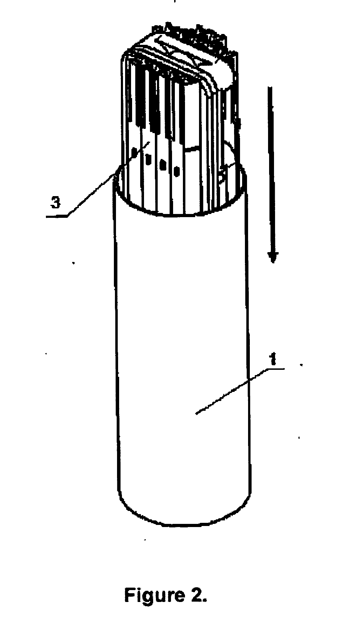

Figure 2 corresponds to a perspective view of the portable system for administering enemas in its storage state.

### Preferred Embodiment of the Invention

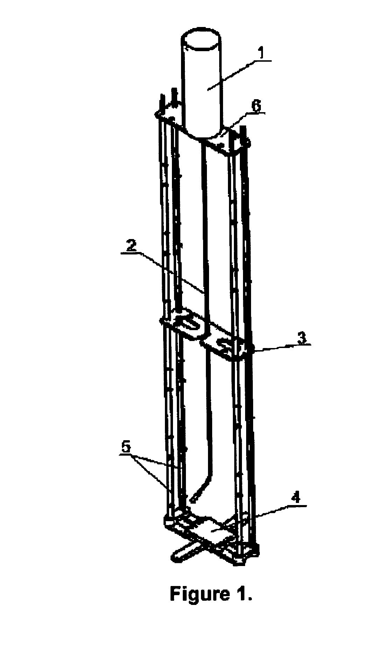

As can be observed in Figure 1, the portable system for administering enemas object of the present invention is designed to work by a difference in pressure. Particularly, the invention comprises a container (1), a hose (2) and a supporting structure (3).

The supporting structure (3), assembled according to Figure 1, has a height, while supporting container (1) which will contain the enema solution, sufficient for there to exist at least the necessary pressure difference for the solution to freely enter the intestine.

Container (1) is designed for the purpose of preparing solutions comfortably and easily, and if previously prepared solutions are used, these can be rebottled easily.

Said hose (2) is connected to container (1) so that the patient can be connected to the system at the distance necessary to achieve the pressure differential required by the system for proper operation.

As can be observed in Figure 1, supporting structure (3) consists of a base (4) capable of providing sufficient stability. Additionally, supporting structure (3) comprises a series of ducts (5) and an upper plate (6), which acts as a support for container (1) and offers rigidity to the enema administering system in its assembled state.

Base (4) is designed such that it can fit inside container (1) when it is dismantled.

The ducts (5) are inserted in the base and between each other. All the ducts have a height equal to or less than the height of the container (1) so that they can be stored inside container (1) when the structure is dismantled, and so that they can reach the height required by the conditions of the user when the structure is assembled.

Upper plate (6) is a plate having a central groove which acts as a guide for both hose (2) and container (1).

## Claims

1. A portable container system for administering enemas, **characterized in that** it consists of a container (1), a hose (2) and a supporting structure (3).

2. A portable container system for administering enemas according to claim 1, **characterized in that** the supporting structure (3), which supports container (1), has a height sufficient for there to exist a necessary pressure difference between the container and the intestine, so that the solution contained in the container can freely enter the intestine.

3. A portable container system for administering enemas according to claim 1, **characterized in that** the supporting structure (3) is capable of being dismantled.

4. A portable container system for administering enemas according to claim 1, **characterized in that** all the pieces of structure (3) can fit inside container (1) separately and together.
